# EUROPEAN PATENT SPECIFICATION

(11) **EP 2 325 384 B1**
(45) Date of publication and mention of the grant of the patent: **28.08.2013**
(21) Application number: 09014637.4
(22) Date of filing: 24.11.2009
(51) Int. Cl.: D06M 10/02, D06M 15/00, D06M 15/285, D06M 23/00

(54) **Permanently hydrophilic nonwoven**
Permanent hydrophiles Vlies
Non tissé hydrophile de façon permanente

(43) Date of publication of application: 25.05.2011
(73) Proprietor: Fibertex Personal Care A/S, 9220 Aalborg Øst (DK)
(72) Inventor: Broch-Nielsen, Thomas, 9220 Aalborg ø (DK); Bøndergaard, Jens, 8600 Silkeborg (DK); Besenbacher, Flemming, 8210 Aarhus V (DK); Kingshott, Peter, 8660 Skanderborg (DK)
(74) Representative: Laufhütte, Dieter

(56) References cited:
- WO-A1-96/18498
- WO-A1-03/064754
- WO-A1-2006/015080
- WO-A2-2008/131152
- US-A1- 2007 054 577

## Description

This invention relates to a process for coating a polymer nonwoven comprising the steps of corona assisted treatment of the surface of the nonwoven to obtain a charged and/or polar surface and formation of a coating layer comprising applying poly(acrylamide-co-diallyldimethylammonium chloride) to the charged and/or polar surface. The invention also relates to a nonwoven fabric obtainable by said process, as well as to the application of such a nonwoven fabric in disposable hygiene products.

In the state of the art it is common to employ nonwoven fabrics in disposable absorbent articles, especially hygiene articles such as diapers, adult incontinence products and feminine hygiene products. The nonwoven fabrics are preferably employed therein as top sheet material or core wrapping material and assist to collect and retain bodily fluids such as urine deposited in the hygiene article. For these applications in hygiene articles it is sometimes necessary to have a hydrophilic nonwoven, i. e. a nonwoven that is easily permeated by aqueous fluids and/or easily charged therewith. However, commonly used polymer nonwoven fabrics, such as for example polypropylene, polyethylene or polyethyleneterephtalate based ones are hydrophobic.

To address this problem different methods for making the surface of a nonwoven hydrophobic have been developed and are known from the state of the art, one of which being topical treatments such as coatings. Inter alia WO 93/04113 or WO 95/25495 describe such methods. Surface coatings can be based on surfactants that typically present themselves as molecules that are hydrophilic on one and hydrophobic on the other end. When applied to a hydrophobic surface such as the surface of polyolefines they orient themselves with the hydrophobic side facing towards and the hydrophilic side facing away from the surface, causing hydrophilic properties. Current state of the art in topical surface treatment is represented by the application of commercial spin-finishes by kiss roll deposition, spray techniques spr3yin, or similar application processes. Commercial spin-finishes commonly used for nonwovens in hygiene industry include products as Stantex S6327 by Pulcra Chemicals and PHP 90 by Schill & Seilacher.

One drawback of these methods is that the associated surfactants and/or other coatings are easily washed off when the nonwoven is exposed to the liquid. As such liquid strike through values are increased with every gush of liquid the nonwoven is exposed to, because the coating substance is not connected to the surface of the nonwoven firmly enough and easily washed off.

Another known method to make the surface of an apolar polymer hydrophilic is post-modification of the surface using corona and plasma treatment, where molecules from the air or another ionizable gas are ionized by an electric field and bind readily with the surface of a polymer like a polyolefin. Corona treatment makes surfaces both hydrophilic and more receptive to bonding with other substances, coatings and adhesives. US 6,118,218 discloses a method for producing plasma suitable for this application. A common disadvantage of corona and plasma treatment is similar to simple surface modification that the molecules grafted to the surface have a tendency to migrate and vanish over time, causing low coating durability upon storage of the material. Hydrophilicity decreases over time. To overcome these disadvantages, EP 1 403 419 suggests to modify at least parts of the fibers used in a nonwoven fabric by chemical grafting with hydrophilic monomer molecules and a radical polymerisation initiator to obtain hydrophilic polymers chemically grafted to the fibers.

State of the art also teaches the addition of hydrophilic additives into the polymer matrix to achieve the desired effect. These addidives preferably migrate to the surface of a fiber to donate hydrophilic properties there. Polymers with hydrophilic additives have the disadvantage of being expensive and time-consuming to manufacture. Intrinsically hydrophilic polymers suitable for the manufacture of nonwovens are also known. For example, US 4,163,078, US 4,257,909 and US 4,810,449 disclose hydrophilic filaments of fibers made by solution spinning of acrylonitrile copolymers. However, solution spinning is relatively expensive and requires organic solvents which are a potential environmental hazard. Melt-extruded hydrophilic fibers and filaments are known but normally expensive and uncommon. An example of obtaining melt-extruded mulitcomponent strands to obtain permanently hydrophilic polymer nonwovens is laid out in EP 0 597 224, whereby one of the components is for example a polymer or copolymer formed by hydrophilic monomers or blocks like polyethylene oxide diamine and/or ethylene acrylic acid.

It is desirable to construct nonwovens that are more hydrophilic and permeable towards polar liquids than common polyolefin based hydrophobic polymer nonwovens employing pure polypropylene, polyethylene and/or polyethylene terephtalate (with surface tensions of 29,4 to 30,1 mN/m, 33,7 to 33,8 mN/m, and 44,6 mN/m, respectively [all values from The Polymer Handbook, 4th edition, volume 2, J. Brandrup, E.H. Immergut 1999]) because neither of these can be penetrated by water, whose surface tension is about 72 mN/m. It is also desirable to overcome the drawbacks from the state of the art and being able to cheaply produce coated nonwovens that are more stable towards repeated exposure to liquids by utilizing the strength of polar bonds. This improves (decreases) the strike through time after repeated exposure to liquids in reducing the undesired solvation of surfactants, and also improves shelf life. Such a process should preferably be easily incorporable in a continuous or semicontinuous production environment that does not require expensive starting materials, as such keeping the cost of the overall production low.

WO 2008/131152 A1 discloses a process of treating the surface of a fibrous article such as a nonwoven. The treatment comprises deposition of a glass film coating followed by oxygen plasma treatment and the subsequent application of a polyelectrolyte layer. The polyelectrolytes comprise oligomers or polymers having a plurality of charged or ionisable sites.

WO 03/064754 A1 discloses a method of hydrophilizing the surface of a synthetic and hydrophobic nonwoven for disposable absorbent articles. The method comprises applying a high energy surface treatment, followed by the application of for example hydrophilic polymers.

WO 96/18498 A1 discloses a method for hydrophilizing a polymeric substrate such as for example a porous nonwoven upon formation of an ionic polymeric layer and application of a polyelectrolyte coating.

US 2007/0054577 A1 discloses electroconductive fabrics, for example electroconductive nonwoven fabrics. The electroconductivity is imposed upon application of a conductive coating with the use of a layer-by-layer technique. The fabric is first pretreated for making it suitable for application of the coating.

WO 2006/015080 A1 discloses a method for durably improving performance properties of fabrics, including for example hydrophilicity. The method comprises two steps. First, a charged surface is provided on the substrate by means of for example application of charged molecules or plasma treatment. Second, a polyelectrolyte is applied.

It is a goal of the present invention to provide an improved method of hydrophilizing the surface of a hydrophobic nonwoven.

This is achieved by the process of claim 1. Such a process for coating a preferably hydrophobic polymer nonwoven comprises the steps of
a) corona assisted treatment of the surface of the nonwoven to obtain, in average, a negatively charged surface, wherein the corona energy is between 1 kJ/m² and 13 kJ/m² and the speed of the nonwoven through the corona discharge is between 1 m/min and 1000 m/min; and
b) formation of a first coating layer comprising applying poly(acrylamide-co-diallyldimethylammonium chloride) to the charged surface.

Polar bonds between the polymer material of the nonwoven fibers and the poly(acrylamide-co-diallyldimethylammonium chloride) coating are consequently introduced. Using poly(acrylamide-co-diallyldimethylammonium chloride) as a coating has the advantage of potentially providing multiple binding sides along its stretch, which can make the coating more chemically and mechanically stable towards repeated exposure to liquids compared to e.g. surfactants from the state of the art with one or few binding points. The straightforward procedure of corona treatment and simple coating can, however, still be retained and the process can in one embodiment easily and economically be performed semi-continuously or continuously. In another embodiment, also discontinuous use is possible.

The above layers can also consist of the poly(acrylamide-co-diallyldimethylammonium chloride) or to a large part consist of poly(acrylamide-co-diallyldimethylammonium chloride). A large part means in this context that the coating comprises at least 50% by weight, preferably at least 65 % by weight, or even more preferably at least 80 % by weight of poly(acrylamide-co-diallyldimethylammonium chloride). Other components can comprise other charged polymeric and/or non polymeric molecules, uncharged molecules and other wanted or unwanted contaminants and additives.

Poly(acrylamide-co-diallyldimethylammonium chloride) is used for coating the corona treated nonwovens in order to form electrostatic bonds between the polar/charged nonwoven surface groups and the coating. Polar/electrostatic binding of the poly(acrylamide-co-diallyldimethylammonium chloride) coating to the nonwoven surface results in a more stable and permanent coating as compared to previous technologies. This can improve (decrease) the strike through time after repeated insults with liquid and reduce the wash-out. By reducing the wash-out, e.g. the amount of washed-off coating in contact with skin ant other surrounding materials is reduced.

In another preferred embodiment a layer is formed by application of a liquid solution of poly(acrylamide-co-diallyldimethylammonium chloride) to the physically and/or chemically modified surface of the nonwoven. One or more of kiss roll deposition, spray techniques, immersion techniques or other techniques suitable to apply the solution to a nonwoven sheet can be used. In a preferred embodiment, kiss roll deposition is employed. In another preferred embodiment the formation of a layer is followed by evaporation of the solvents. Preferred solvents for the application include water, methanol, ethanol and other alcohols, acetone, DMSO and other non-protic polar organic solvents like acetylacetate and other esters, different ethers, as well as combinations thereof.

Nonwoven fabrics suitable for treatment with the process of the above embodiments can comprise or consist of nonwoven fibers made from any polymer common in the art, which has hydrophobic or only slightly hydrophilic properties. In one embodiment the nonwoven comprise or consist of thermoplastic polymers, including polymer compositions, mixtures and blends. Examples of suitable thermoplastic polymers for the use herein include polyolefins, preferably polypropylene or polyethylene or further polyethylene-polypropylene copolymers; polyesters, polyamides; polyhydroxyalkanoates, and mixtures thereof. The fibres may also be multicomponent fibres, including bicomponent fibres. In one embodiment, polypropylene and polypropylene compositions are preferred, including homopolymers and copolymers of propylene. One preferred polymer material is polypropylene linked with the help of a metallocene catalyst. Such metallocene-polypropylene polymers offer a much greater level of control than conservative polypropylene materials that are connected with the help of a Ziegler-Natta catalyst, because the metallocene molecules offer better control towards how the monomers are linked, so that a proper choice of catalysts can produce isotactic, syndiotactic or atactic polypropylene, or even a combination of these.

In one specifically preferred embodiment, suitable materials for the nonwoven to be coated are selected from one or more of polyethylene, polypropylene.

In one embodiment of the invention a suitable nonwoven for coating is manufactured by spunbound/meltblown layering of only one or more spunbound (S) layers and/or one or more meltblown (M) layers, e.g. in an SMMMS, SMMS or SSMMS configuration, and other configurations known to the skilled person.

In another embodiment of the invention a suitable nonwoven for coating is manufactured by spunbound/meltblown layering of only one or more spunbound (S) layers, e.g. in a S, SS or SSS configuration, and other configurations known to the skilled person.

The fibres the nonwoven fabrics or layers in more-layered fabrics herein can in one embodiment be nanofibers, with a diameter of less than 1000 nanometers, or in another embodiment be fibers with a diameter of more than a micrometer, in a yet another embodiment of more than 10 micrometers, or combinations thereof. Alternatively, or in addition, a nonwoven herein may for example comprise or consist of one or more spunbond layers from fibers with an average fiber diameter of, for example, from 6 to 22 microns, or from 8 to 18 microns. The meltblown web or layer herein may for example comprise or consist of one or more meltblown layers from fibers that have an average fiber diameter from 1 to 5 microns, or 1 to 4 microns, or preferably from 1 to 3 microns, or less than 1 micron. Fiber diameters expressed hereinabove for meltblown layers and spunbound layers are interchangeable.

A representative suitable nonwoven template can be made of standard polypropylene and have an area weight of between about 5 and about 20 g/m², preferably between about 8 and about 15 g/m².

In one embodiment the corona and/or plasma treatment is performed in an optimum energy range to prepare the surface in an optimized way for subsequent application and binding of a coating layer. The existence of such a window of optimum corona effect is well known within the industry. In one embodiment, the preferred corona energy is between about 1 kJ/m² and about 7 kJ/m².

All the steps mentioned in the foregoing embodiments can be carried out in a discontinuous, a semicontinuous, or preferably a continuous manner. In an especially preferred embodiment, the steps of plasma and/or corona assisted treatment of the nonwoven fiber surface to introduce charged polar surface species and application of poly(acrylamide-co-diallyldimethylammonium chloride) are carried out in a continuous manner on line.

The present invention further relates to a nonwoven fabric according to claim 5. Such a nonwoven is obtainable by the process described hereinabove. It comprises a coating layer comprising poly(acrylamide-co-diallyldimethylammonium chloride) bound by multiple polar bonds and has an increased surface tension relative to standard nonwoven. This results in an easy break through of aqueous liquids as for example urine through a nonwoven web.

In one embodiment the nonwoven will maintain low strike through values even at a large number of test in a repeated liquid strike through test. As such, the LST can increase by less than 30% after 5, preferably 10, more preferably 20, most preferably 50 insults, in a preferred embodiment by less than 20% or less than 15%, in yet another preferred embodiment by less than 10%, in an especially preferred embodiment by less than 5% and in one most preferred embodiment by less than 1% or even less than 0,5%. In one embodiment, the liquid strike through of a representative 10 g/m² SMMMS PP nonwoven (4,4g / 0,4g / 0,4g / 0,4g / 4,4g) may be less than 7 seconds after 20, preferably 50 gushes, in another embodiment less than 5 seconds after 20, preferably 50 gushes and in an even preferred embodiment less than 3 seconds after 20, preferably gushes. In stark contrast, when using standard finishes a steep increase in values after about 3 to 5 tests is to be expected.

The present invention also relates to the use of a nonwoven fabric according to claim 7. As such, it relates to the application of a nonwoven fabric obtainable by a process according to the invention in a disposable hygiene product. Preferred disposable hygiene products encompass baby diapers, adult incontinence products and feminine hygiene products. In such products, the nonwoven fabric produced by the method according to the present invention may preferably be employed as a top sheet and/or a wrap material.

The following test methods are used to obtain the numerical values set forth in the above description as well as in the claims of this patent application.

### Repeated strike through test:

The standardized test method EDANA/INDA test WSP 70.7 (05) for nonwoven cover stock multiple liquid strike through time using simulated urine was used. In order to increase the number of repeated insults above 5, the filter paper was changed for every 5 insults, e.g. before insult 6, 11, 16, 21 etc.

In the examples, where some values for LST are indicated for a number of repetitions (such as 11 to 20), the value represents the average LST for the respective number of repetitions.

The following examples are set forth for the purpose of illustrating the invention in more detail.

### Comparative Example 1:

In Comparative Example 1 a 10 g/m² spunbound/meltblown SMMMS (4,4g / 0,4g / 0,4g / 0,4 g / 4,4g) polypropylene nonwoven was coated with standard spin-finish (PHP26, Schill & Seilacher) at 100m/min to obtain a standard core wrap nonwoven.

Comparative Example 1 corresponds to a nonwoven fabric coated to become hydrophilic as of the the state of the art.

### Comparative Example 2:

The surface of a 10 g/m² spunbound/meltblown SMMMS (4,4g / 0,4g / 0,4g / 0,4 g / 4,4g) polypropylene nonwoven was physically and chemically modified by corona treatment. The corona energy was set to 4,8 kJ/m² and the speed of the nonwoven through the corona discharge was 100 m/min. The chemical modification therein consists essentially of the addition of polar surface species such as hydroxyl, carboxyl, epoxy and aldehyde groups to the surface of the fibers of the PP nonwoven.

The resulting nonwoven without further treatment corresponds to a corona and plasma treated nonwoven fabric from the state of the art.

### Example 1:

The corona treated polypropylene nonwoven of Comparative Example 2 is used as a basis and a single layer of positively charged poly(acrylamide-co-diallyldimethylammonium chloride) is applied through 100 m/min kiss roll deposition of a 10 mg/ml solution of this polymer in water.

The deposition step is immediately followed by baking for 5 seconds at 100°C in an oven. After baking, the nonwoven is dry and hydrophilic. The obtained nonwoven bears one layer of positively charged poly(acrylamide-co-diallyldimethylammonium chloride) coating, bound on multiple sides through polar bonds. It has in one case been stored for 4 days before measurement, and in another case for 25 days in order to simulate storage of the product before being commercially sold.

Repeated strike through tests of the Comparative Example 1 (10 g/m² SMMMS with standard hydrophilic spin-finish) and the single coated nonwoven of Example 1 both after 4 days aging and 25 days aging have been carried out. The results are shown in Table 1.

**Table 1:**

| Comparison of LST of corona reference with single layer coatings and standard 10 g/m² SMMMS with standard hydrophilic spinfinish. Numbers are in seconds. | | | | | | | | | | | |
|---|---|---|---|---|---|---|---|---|---|---|---|
| Sample/insult number | 1 | 2 | 3 | 4 | 5 | 6 | 7 | 8 | 9 | 10 | 11-20 |
| | | | | | | | | | | | |
| **Comp. ex. 1** | 1,4 | 2,3 | 3,5 | 2,7 | 2,6 | 4,6 | 7,6 | 7,7 | 5,2 | 6,5 | 7,4 |
| **Example 1 (4 days)** | 32,4 | 22,0 | 15,8 | 13,1 | 11,5 | 9,2 | 7,4 | 7,8 | 5,9 | 5,3 | - |
| **Example 1 (25 days)** | 22,1 | 11,6 | 10,3 | 8,7 | 8,7 | 12,2 | 8,4 | 9,3 | 8,2 | 7,3 | 3,7 |

While the conservatively coated nonwoven of the comparative example shows a significant increase in strike through times after the increasing number of liquid insults, the nonwovens of Example 1 show a continuous decrease in strike through times. The desirable effect achieved by means of the process presented hereinabove is clearly proved by Example 1, where the averaged strike through time between 11 and 20 liquid insults is as low as 3,7 seconds for a nonwoven aged 25 days after coating. It can be concluded that no or only insignificant parts of the coating material has been dissolved in the insulting liquid.

### Example 2

In this example a nonwoven is corona treated and coated in an inline continuous process.

The surface of a 10g/m² spunbond/meltblown SMMMS polypropylene nonwoven was physically and chemically modified by corona treatment. Four different settings were used (A, B, C, D).

| Setting nr. | Corona effect [kJ/m²] | Speed [m/min] | Weight [g/m²] | Drying time [s] |
|---|---|---|---|---|
| A | 4,8 | 100 | 0,1 | 4,8 |
| B | 9,6 | 50 | 0,055 | 9,6 |
| C | 9,6 | 50 | 0,35 | 9,6 |
| D | 2,4 | 200 | 0,22 | 2,4 |

The chemical modification in all cases consists essentially of the addition of polar surface species such as hydroxyl, carboxyl, epoxy and aldehyde groups to the surface of the fibers of the PP nonwoven.

The corona treated polypropylene nonwoven was then immediately (inline) used as a basis for binding of positively charged polymers to the nonwoven. Coating with a single layer of positively charged poly(acrylamide-co-diallyldimethylammonium chloride) was achieved through kiss roll deposition of a 40 mg/ml solution of this polymer in water.

The deposition step was immediately followed by drying at 100°C in an oven. After drying, the nonwoven is dry and hydrophilic. The obtained nonwoven bears one layer of positively charged polymer coating, bound on multiple sides through polar bonds.

### Results:

The coated nonwovens were tested using the repeated liquid strike-through test with 50 repetitions. There was no obvious decrease or increase in strike-through time after 50 measurements.

Strikethrough data from sample A, B, C, and D are shown in Table 2. Corona references without coating are also shown for each sample.

**Table 2**

| The repeated strike-through data in seconds. | | | | | | | | | | |
|---|---|---|---|---|---|---|---|---|---|---|
| Sample/insult number | 1 | 2 | 3 | 4 | 5 | 6-10 | 11-20 | 21-30 | 31-40 | 41-50 |
| **Corona reference, A** | >100 | - | - | - | - | - | - | - | - | - |
| **Coated sample, A** | 5,1 | 2,7 | 2,6 | 2,2 | 2,0 | 2,2 | 2,1 | 2,0 | 2,1 | 2,2 |
| **Corona reference, B** | >100 | - | - | - | - | - | - | - | - | - |
| **Coated sample, B** | 2,6 | 2,5 | 2,5 | 2,4 | 2,2 | 2,5 | 2,2 | 2,4 | 2,3 | 2,6 |
| **Corona reference, C** | >100 | - | - | - | - | - | - | - | - | - |
| **Coated sample, C** | 1,5 | 2,1 | 2,0 | 2,0 | 1,8 | 2,1 | 2,2 | 2,1 | 2,3 | 2,1 |
| **Corona reference, D** | >100 | - | - | - | - | - | - | - | - | - |
| **Coated sample, D** | 3,7 | 3,7 | 3,4 | 3,1 | 2,7 | 3,1 | 3,1 | 3,2 | 3,0 | 3,2 |

### Example 3

In this example a nonwoven is corona treated and coated in an inline continuous process.

The surface of a 15g/m² spunbond SSS polypropylene nonwoven was physically and chemically modified by corona treatment. The corona energy was set to 4,80kJ/m² and the speed of the nonwoven through the corona discharge was 100m/min. The chemical modification therein consists essentially of the addition of polar surface species such as hydroxyl, carboxyl, epoxy and aldehyde groups to the surface of the fibers of the PP nonwoven.

The corona treated polypropylene nonwoven was then immediately (inline) used as a basis for binding of positively charged polymers to the nonwoven. Coating with a single layer of positively charged poly(acrylamide-co-diallyldimethylammonium chloride) was achieved through 100 m/min kiss roll deposition of a 40 mg/ml solution of this polymer in water. The weight of coating was 0,22g/m².

The deposition step was immediately followed by drying for approximatly 2,4 seconds at 100°C in an oven. After drying, the nonwoven is dry and hydrophilic. The obtained nonwoven bears one layer of positively charged polymer coating, bound on multiple sites through polar bonds.

### Results:

The coated nonwovens were tested using the repeated liquid strike-through test with 50 repetitions. There was no obvious decrease or increase in strike-through time after 50 measurements.

**Table 3**

| The repeated strike-through data in seconds. Corona reference strike through times were only measured to 10 insults. | | | | | | | | | | |
|---|---|---|---|---|---|---|---|---|---|---|
| Sample/insult number | 1 | 2 | 3 | 4 | 5 | 6-10 | 11-20 | 21-30 | 31-40 | 41-50 |
| **Corona reference** | 8,0 | 5,2 | 6,1 | 6,2 | 5,5 | 7,22 | - | - | - | - |
| **Coated sample** | 3,1 | 3,5 | 3,8 | 4,2 | 2,7 | 3,4 | 3,3 | 3,2 | 3,1 | 3,1 |

## Claims

1. A process for coating a polymer nonwoven comprising the steps of:
a) corona assisted treatment of the surface of the nonwoven to obtain, in average, a negatively charged surface, wherein the corona energy is between 1 kJ/m² and 13 kJ/m² and the speed of the nonwoven through the corona discharge is between 1 m/min and 1000 m/min; and
b) formation of a coating layer comprising applying poly(acrylamide-co-diallyldimethylammonium chloride) to the charged surface.

2. The process of claim 1, wherein the poly(acrylamide-co-diallyldimethylammonium chloride) is applied as a liquid solution thereof, preferably followed by evaporation of the solvent.

3. The process of any preceeding claim, wherein the nonwoven fibers are made from one or more polyolefins, preferably selected from PP and PE.

4. A nonwoven fabric obtainable by the process of any preceeding claim, wherein the nonwoven comprises a coating layer comprising poly(acrylamide-co-diallyldimethylammonium chloride) bound by multiple polar bonds and wherein the nonwoven fabric preferably has an average 40 th to 50^{th} repeated liquid strike through value below 10 seconds (EDANA/INDA test WSP 70.7-05).

5. The nonwoven fabric of claim 4, wherein the liquid strike through time of the nonwoven increases by less than 15 % after 50 insults in a repeated liquid strike through test (EDANA/INDA test WSP 70.7-05).

6. Use of a nonwoven fabric according to claim 4 or 5 in a disposable hygiene product, preferably in baby diapers, adult incontinence products and/or feminine hygiene products, preferably as topsheet and/or wrap material.

## Patentansprüche

1. Verfahren zum Beschichten eines Polymervlieses, mit den folgenden Schritten:
a) koronagestützte Behandlung der Vliesoberfläche, um im Durchschnitt eine negativ geladene Oberfläche zu erhalten, wobei die Koronaenergie zwischen 1 kJ/m² und 13 kJ/m² beträgt und die Geschwindigkeit des Vlieses durch die Koronaentladung zwischen 1 m/min und 1000 m/min beträgt; und
b) Bilden einer Überzugsschicht, wobei auf die geladene Oberfläche Poly(acrylamid-co-diallyldimethylammoniumchlorid) aufgebracht wird.

2. Verfahren nach Anspruch 1, wobei das Poly(acrylamid-co-diallyldimethylammoniumchlorid) als flüssige Lösung davon aufgebracht wird, vorzugsweise mit anschließender Verdampfung des Lösungsmittels.

3. Verfahren nach einem der vorhergehenden Ansprüche, wobei die Vliesfasern aus einem oder mehreren Polyolefinen bestehen, die vorzugsweise aus PP und PE ausgewählt sind.

4. Vliesstoff, den man mit dem Verfahren nach einem der vorhergehenden Ansprüche erhalten kann, wobei das Vlies eine Überzugsschicht aus Poly(acrylamid-co-diallyldimethylammoniumchlorid) umfasst, das durch eine Vielzahl polarer Bindungen gebunden ist, und wobei der Vliesstoff vorzugsweise einen Durchschnittswert für die Flüssigkeitspenetration bei der 40. bis 50. Wiederholung von unter 10 Sekunden (EDANA/INDA-Test WSP 70.7-05) besitzt.

5. Vliesstoff nach Anspruch 4, wobei die Zeit für die Flüssigkeitspenetration des Vlieses nach 50 Versuchen bei einem Test mit wiederholter Flüssigkeitspenetration (EDANA/INDA-Test WSP 70.7-05) um weniger als 15% zunimmt.

6. Verwendung eines Vliesstoffes nach Anspruch 4 oder 5 bei einem einmal verwendbaren Hygieneprodukt, vorzugsweise bei Babywindeln, Inkontinenzprodukten für Erwachsene und/oder Damenhygieneprodukten, vorzugsweise als Deckschicht und/oder Umhüllungsmaterial.

## Revendications

1. Procédé de revêtement d'un non-tissé polymère, comprenant les étapes de :
a) un traitement assisté corona de la surface du non-tissé pour obtenir, en moyenne, une surface chargée négativement, où l'énergie corona est entre 1 kJ/m² et 13 kJ/m², et la vitesse du non-tissé à travers la décharge corona est entre 1m/min et 1000 m/min ; et
b) la formation d'une couche de revêtement comprenant l'application de poly(acrylamide-co-diallyldiméthylammonium chlorure) à la surface chargée.

2. Procédé selon la revendication 1, dans lequel le poly(acrylamide-co-diallyldiméthylammonium chlorure) est appliqué comme une solution liquide de celui-ci, de préférence suivi de l'évaporation du solvant.

3. Procédé selon l'une quelconque des revendications précédentes, dans lequel les fibres non-tissées sont réalisées à partir d'une ou de plusieurs polyoléfines, de préférence sélectionnées parmi PP et PE.

4. Tissu non-tissé pouvant être obtenu par le procédé selon l'une quelconque des revendications précédentes, où le non-tissé comprend une couche de revêtement comprenant poly(acrylamide-co-diallyldiméthylammonium chlorure) lié par des liaisons polaires multiples, et où le tissu non-tissé a de préférence une valeur moyenne de résistance à la pénétration des liquides de 40 à 50 en dessous de 10 secondes (EDANA/INDA test WSP 70.7-05).

5. Tissu non-tissé selon la revendication 4, dans lequel le temps de résistance à la pénétration des liquides du non-tissé augmente selon moins que 15% après 50 affronts dans un test de résistance à la pénétration des liquides répété (EDANA/INDA test WSP 70.7-05).

6. Utilisation d'un tissu non-tissé selon la revendication 4 ou 5 dans un produit d'hygiène jetable, de préférence dans les couches pour bébés, des produits pour l'incontinence des adultes et/ des produits pour l'hygiène de la femme, de préférence comme feuille de dessus et/ou comme matériau d'enveloppement.
